# EUROPEAN PATENT SPECIFICATION

(11) **EP 1 467 710 B1**
(45) Date of publication and mention of the grant of the patent: **06.08.2008**
(21) Application number: 02795221.7
(22) Date of filing: 18.12.2002
(51) Int. Cl.: A61K 9/113, A61K 9/18

(54) **PHARMACEUTICAL COMPOSITION COMPRISING AN OIL/WATER/OIL DOUBLE MICROEMULSION INCORPORATED INTO A SOLID SUPPORT**
PHARMAZEUTISCHE ZUSAMMENSETZUNG ENTHALTEND EINE WASSER/ÖL/WASSER DOPPELTE MIKROEMULSION IN EINEM FESTEN TRÄGER
COMPOSITION PHARMACEUTIQUE COMPRENANT UNE DOUBLE MICRO-EMULSION HUILE/EAU/HUILE INCORPOREE DANS UN SUPPORT SOLIDE

(30) Priority: 19.12.2001 IT MI20012694
(43) Date of publication of application: 20.10.2004
(73) Proprietor: Geotech Pharma Inc., Toronto, M5H 3S5 (CA)
(72) Inventor: CARLI, Fabio, I-34136 Trieste (IT); CHIELLINI, Elisabetta, I-56126 Pisa (IT)
(74) Representative: Gervasi, Gemma
(86) International application number: PCT/EP2002/014472
(87) International publication number: WO 2003/051334

(56) References cited:
- WO-A-00/09093
- WO-A-90/03164

## Description

### PRIOR ART

The hydrophobic characteristics and consequently low water solubility of many drugs make it such that their oral bioavailability is low or however non optimal. Many approaches have been proposed and also realised in the past in an attempt to overcome this problem, such as for example micronisation, the inclusion in complexing agents such as cyclodextrine, co-precipitation with linear hydrophilic polymers etc.

In the last few years, a line of approach which has shown itself to be particularly fruitful is that based on the use of oils, essentially constituted by mixtures of mono-, di-, and tri-glycerides, associated with surfactants and/or with a hydrophilic phase, such as for example esters of propyleneglycols or glycerol.

In this case the poorly bio-available drug is dissolved in the above described mixtures and the resultant solution is usually introduced into a capsule of soft gelatine.

This oil-based approach has been further developed with appropriate systems, formulated such that when dispersed in water, spontaneously form emulsions or microemulsions (WO 99/29300, US 5,993,858), in which the degree of fine dispersion results in a further potential improvement in bioavailability.

Because, in this system, the drug is dissolved in a liquid phase, further improvement studies have attempted to transform such liquid phases into dry solid powders so as to allow a greater workability in terms of industrial processes with the attainment of a solid pharmaceutical form, such as a pill or a capsule. For example, the incorporation of oil/surfactant mixtures into cross-linked polymers has been described in the patent EP 0598337, whilst auto-emulsifying compositions have been solidified through their entrapment into gelifying linear polymers such as cellulose derivatives (Proceed. lnt'l Symp. Control.Rel. Bioact. Mater. 27(2000), # 6209).

WO 90/03164 A and WO 00/09093 A disclose microemulsions absorbed an solid carriers.

Notwithstanding the numerous studies addressed at the achievement of improved compositions, the compositions in the known art display unsatisfactory solubility and velocity of dissolution characteristics.

### SUMMARY

Now we have surprisingly found that when a poorly hydrosoluble drug is added to a double microemulsion oil/water/oil (o/w/o) and this is incorporated into a solid support constituted by a microporous inorganic substance or by an adsorbent colloidal inorganic substance or by a cross-linked swellable in water polymer, unexpected improvements in the solubility and the velocity of dissolution of the drug with respect to the previously described compositions are obtained.

For this reason, the present invention relates to a pharmaceutical composition in the form of powders or microgranules, comprising an oil/water/oil double micro emulsion incorporated into a solid support constituted by a microporous inorganic substance or by an adsorbent colloidal inorganic substance or by a cross-linked swellable in water polymer, with the drug dissolved or dispersed in one or more of the three phases of the double microemulsion.

### BRIEF DESCRIPTION OF THE FIGURES

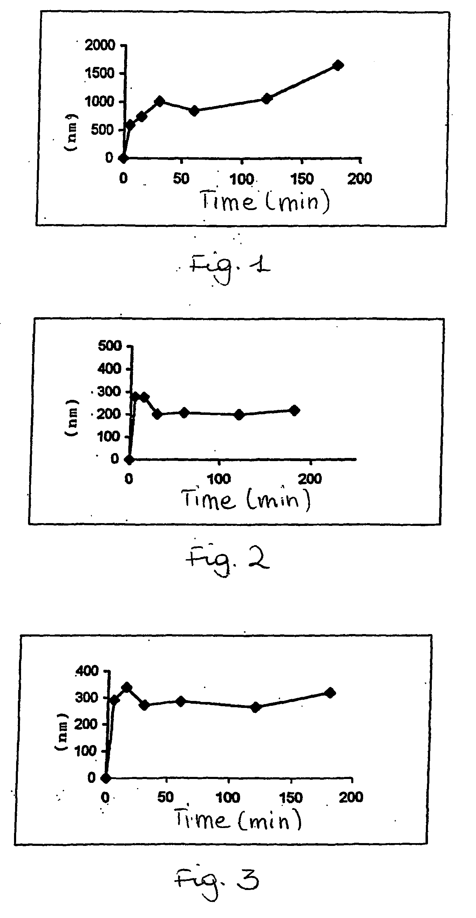

Figures 1, 2 and 3 represent the size of the oil microdrops released from the compositions in the examples N°. 2, 3, and 4, in buffered solution at pH 7.5 at 37°C.

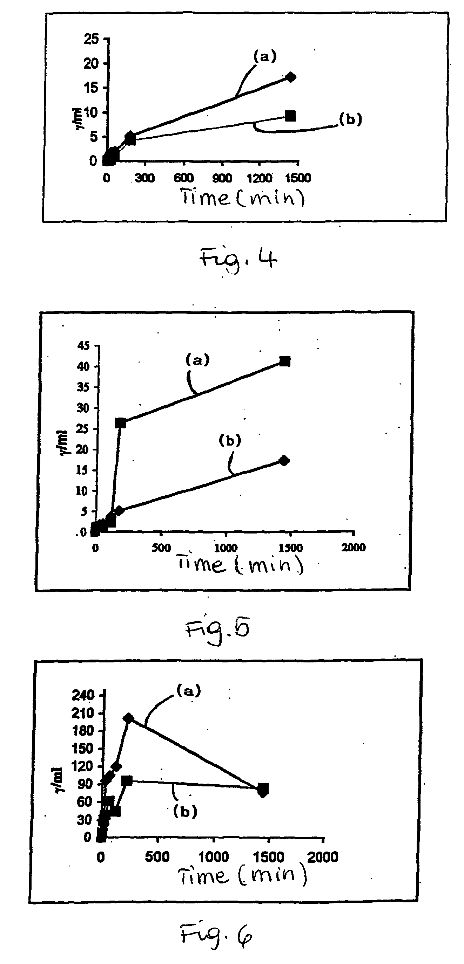

Figure 4 shows the solubilisation kinetics data ("non sink") of the cyclosporin released from the composition in example N° 2 (curve (a)) (o/w/o double microemulsion on colloidal silica) in comparison to cyclosporin as such (curve (b)), in buffer at pH 7.5, at 37°C.
Figure 5 shows the solubilisation kinetics data ("non sink") of cyclosporin from the composition in example N° 1 (curve (a)) (o/w/o double microemulsion incorporated into microporous silica) in comparison to cyclosporin as such (curva (b)), in buffered solution at pH 7.5, at 37°C.
Figure 6 shows the solubilisation kinetics data ("non sink ") of cyclosporin released from the composition in example N° 3 (curve (a)) (o/w/o double microemulsion incorporated into cross-linked polyvinylpyrrolidone) in comparison to the cyclosporin released from a simple oil/surfactant mixture in cross-linked polyvinylpyrrolidone (composition in example N° 4) (curve (b)), in buffer at pH 7.5 at 37°C.

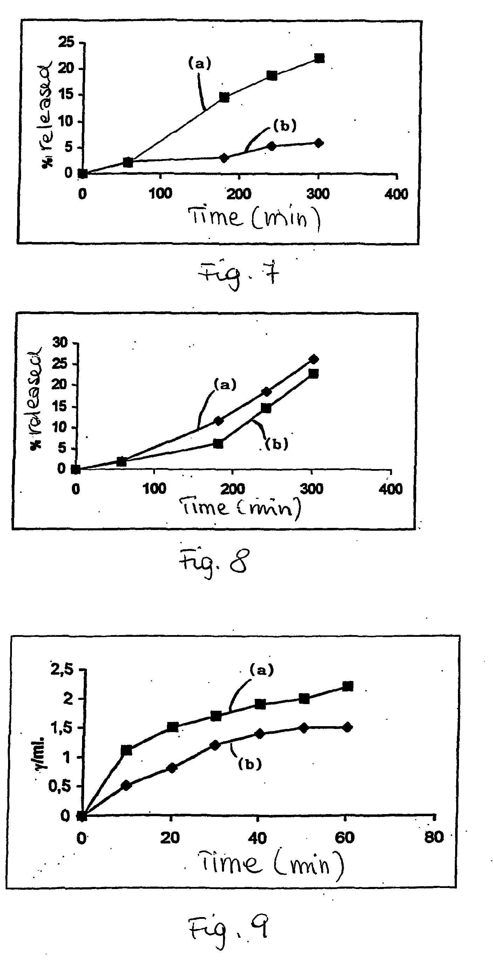

Figure 7 shows the dissolution velocity data ("sink") of cyclosporin released from the composition in example N° 3 (curve (a)), in comparison to the cyclosporin as such (curve (b)) in buffer at pH 7.5 , at 37°C.
Figure 8 shows the dissolution velocity data ("sink") of the cyclosporin released by the composition in example 3 (curve (a)) (o/w/o double microemulsion incorporated into cross-linked polyvinylpyrrolidone) in comparison to the simple oil/surfactant mixture loaded onto cross-linked polyvinylpyrrolidone with the composition in example N° 4 (curve (b)), in buffer at pH 7.5 at 37°C..
Figure 9 shows the dissolution velocity ("sink") of hydrocortisone acetate from the composition in example N° 6 (curve (a)) (o/w/o double microemulsion incorporated into microporous silica) in comparison with the simple o/w microemulsion incorporated into microporous silica (composition in example N° 7) (curve (b)), in buffer at pH 5.5 at 37°C.

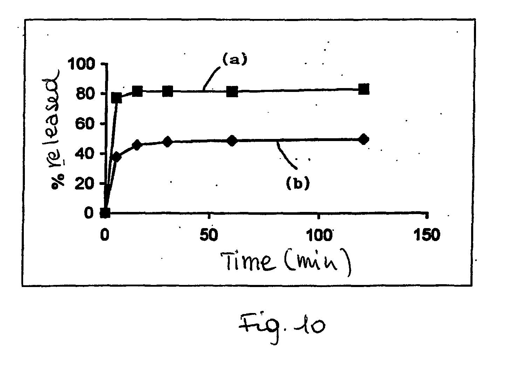

Figure 10 shows the dissolution velocity data of ubidecarenone released from the composition in example N° 8 (curve (a)) (o/w/o double microemulsion incorporated into cross-linked polyvinylpyrrolidone) in comparison to the simple oil/surfactant microemulsion incorporated into the same polymer (composition of example N° 9), in buffer at pH 7.5 added to sodium lauryl sulphate, at 37°C.

### DETAILED DESCRIPTION OF THE INVENTION

The characteristics and the advantages of the pharmaceutical compositions according to the present invention comprising an oil/water/oil o/w/o double microemulsion incorporated into a solid support will be outlined in the following detailed description.

The preparation process of the compositions according to the present invention can be outlined as follows, essentially distinguishing two main stages, i.e. the preparation of the double microemulsion and the incorporation of that double microemulsion onto a solid support.
1. The o/w/o double microemulsion according to the present invention is prepared according to a process comprising the following stages:
   a) dissolution of the drug in an oil or in a mixture of oils;
   b) addition of the oil solution of stage a) to water or to an aqueous solution;
   c) addition of surfactant and optionally of cosurfactant to the mixture of stage b) and agitation with the formation of the o/w microemulsion;
   d) addition of the o/w microemulsion of stage c) to an oil or to a mixture of oils optionally containing drug and/or surfactant and/or cosurfactant and agitation with the formation of the o/w/o double microemulsion.
2. The o/w/o double microemulsion of point 1 is incorporated into a solid support in powder form, slowly adding said microemulsion to said solid support kept under constant mixing/agitation, using equipments such as high mixing efficiency granulators, extruders or fluid bed granulators.

One obtains the support/double microemulsion composition in the form of a powder or of microgranules which can be sieved to eliminate the aggregates.

Possible variations to said process can be contrived in response to different objectives, amongst these that of increasing by as much as possible the amount of drug incorporated.

For example, during the double microemulsion preparation phase, the drug can be dissolved not just in the internal oil phase of the first microemulsion (stage (a) of point 1) but also in the oil or in the mixture of stage (d).

A further variation is that of preliminarily loading the drug onto the solid support, for example dissolving it in water or in an appropriate mixture of solvents and adding the solution to the solid support, with later drying to eliminate the water and/or the solvent components of the mixture. To the resulting drug/solid support powder is then added the double microemulsion prepared as in point 1, according to the addition method described in point 2.

According to a further variation, the oil phase, besides the dissolved drug it contains also drug in suspended form so that the double microemulsion at the moment of incorporation into the solid support contains also solid drug particles.

Unexpectedly, also in this case the clearly improved properties of passing into solution with respect to the drug as such are maintained.

In any case, for all the variations of the process described above, the final characteristics of the compositions obtained do not vary substantially from those obtainable by following the primary process, as well as further minor variations to the process herein described can be introduced maintaining the final characteristics of the compositions constant.

From the description of the process it appears that the o/w/o double microemulsion is constituted of oil, water, surfactant, cosurfactant and that the internal oil phase, and optionally also the external oil phase, contain the dissolved or suspended drug.

Such double microemulsion has the following composition by weight.

| | |
|---|---|
| oil (internal phase) | from 1.5% to 3.0% |
| water or aqueous solution | from 6.1 % to 10.0% |
| Surfactant | from 2.4% to 5.0% |
| Cosurfactant | from 0.0% to 2.0% |
| oil (external phase) | from 80.0% to 90.0% |

Preferred drugs for the present invention are drugs soluble in oil and sparingly soluble in biological aqueous fluids, particularly drugs with low polarity. Non-exhaustive examples of these drugs are megestrol acetate, hydrocortisone acetate, ubidecarenone, lovastatin, cyclosporin, pyroxicam, nifedipine, isoflavone, temazepam, carbamazepine, glibenclamide, progesterone and ibuprofen.

The oil used for the microemulsion is an oil of plant origin such as olive oil, soya oil, corn oil and coconut oil; or an oil of synthetic origin, such as isopropyl myristate, isopropyl palmitate, ethyl laurate etc.

Short, medium or long chain fatty acids and mixtures of mono-, di-, and tri-glycerides of plant, synthetic or semi-synthetic origin and their esterified derivatives, for example with polyethylene glycols may also be used.

The surfactants can be of natural or synthetic origins. One can also use combinations of surfactants with different characteristics. Non-exhaustive examples of surfactants particularly suited to the present invention are surfactants having the trade names of: Tween^{®} , Brij^{®}, Span^{®}, Myrj^{®}, Poloxamer^{®} etc.

The cosurfactants can be of synthetic origins such as for example short chain alcohols such as ethanol, isopropanol , etc., or of natural origins, such as for example phosphatidylcholine, phosphatidylethanolamine, phosphatidylglycerol and derivatives thereof.

The aqueous phase of the double microemulsion can be constituted by demineralised water or by an acidic or basic aqueous solution or by an aqueous buffer solution. The solid support into which the double microemulsion is incorporated is selected from the group constituted by microporous inorganic substances, colloidal inorganic adsorbent substances with high surface area or by cross-linked swellable in water polymers. The microporous inorganic substances are selected from the group comprising silica, silicates, zeolites, alumina, activated carbon, etc.

The colloidal inorganic adsorbent substances are selected for example from colloidal silica, magnesium trisilicate, argil, magnesium hydroxide, talc, etc.

The cross-linked reswellable in water polymers are selected from the group comprising cross-linked polyvinylpyrrolidone (crospovidone), cross-linked sodium carboxymethystarch, cross-linked sodium carboxymethylcellulose, cross-linked polystyrene and cross-linked polymethylmetacrylate.

The weight ratio between the o/w/o double microemulsion and the solid support is comprised of between 1:100 and 25:1, preferably between 1:2 and 5:1.

The amount of drug in the final composition is comprised of between 0.001 % and 75% by weight with respect to the total weight of the final composition and preferably between 0.01 % and 30%.

The compositions of the present invention display unexpectedly improved characteristics regarding the bioavailability of the drugs, in particular:
a) The sizes of the oil microdrops released by the solid support in an aqueous environment are less than 1 micrometer or however close to that limit.
b) The velocity of dissolution ("sink") determined in appropriate aqueous buffer at physiological pH is superior to that attainable with oil/surfactant mixtures or with simple microemulsions.
c) The solubilisation kinetics ("non sink") determined in aqueous buffer at physiological pH is superior to the kinetics attainable with oil/surfactant mixtures or with simple microemulsions.

For clarity of interpretation it should be underlined that the meanings of "sink" and "non sink" are as follows:

To conduct a dissolution test in "sink" conditions means to introduce into the medium of dissolution a quantity of sample such that the concentration of the main ingredient is less than 20% of the maximum solubility of the drug in the same medium of dissolution.

Instead to conduct a dissolution test in "non sink" conditions means to introduce into the medium of dissolution a quantity of sample equal to at least 50 - 100 times the solubility of the drug in the medium of dissolution.

The tests carried out in "sink" conditions emphasise the velocity of dissolution, whilst the tests carried out in "non sink" conditions emphasise the saturation concentration of the main ingredient at equilibrium.

The compositions object of the present invention are formulated for therapeutic use in capsules, pills, packets, suspensions, etc. with the appropriate addition of pharmaceutically acceptable excipients or diluents.

Some examples of preparations and the characterisation tests of the compositions obtained are reported herein.

### Example N°1

| Components of the o/w/o microemulsion | Quantity (g) |
|---|---|
| Cyclosporin | 2.89 |
| Akoline^{®}(internal phase) | 0.96 |
| Demineralised water | 2.69 |
| Tween 80^{®} | 1.05 |
| Akoline^{®} (external phase) | 29.69 |

| | |
|---|---|
| Akoline^{®} is a mixture of mono- and di-glycerides supplied by the company Karshamns. | |

An o/w microemulsion was first of all prepared by magnetically stirring Akoline^{®} (0.96g) containing dissolved cyclosporin (0.29g), demineralised water (2.69g) and Tween 80^{®} (1.05g), at a temperature of 25°C and maintaining the agitation at a velocity of 500 rpm for 1 hour.

The o/w microemulsion thus obtained has then been added using magnetic stirring at a speed of 500 rpm, to a solution of cyclosporin (2.60g) in Akoline^{®} (29.69g) and the agitation has been maintained for 2 hours, obtaining in this manner an o/w/o double microemulsion containing cyclosporin.

The microemulsion thus obtained has been incorporated into microporous silica Syloid^{®} by granulation in a high efficiency granulator from Società Battagion (Bergamo-Italy).

The weight ratio between the o/w/o microemulsion and the silica was 2.5:1.0.

The composition obtained was in the form of a powder, having good flow characteristics and uniform granulometry.

### Example N° 2

An o/w/o double microemulsion has been prepared as in example 1.

The microemulsion has then been incorporated into microporous silica Aerosil 300^{®} by granulation in a high efficiency granulator from Società Battagion (Bergamo-Italy).

The weight ratio between the o/w/o microemulsion and the silica Aerosil 300^{®} was of 2.5:1.0.

The composition obtained was in the form of a powder having good flow characteristics and uniform granulometry.

### Example N° 3

An o/w/o double microemulsion has been prepared as in example 1.

The microemulsion has then been incorporated into the cross-linked polymer crospovidone Kollidon CL^{®} in the weight ratio 1.2:1.0 in a twin screw extruder from APV Company (U.K.) and the product obtained has been spheronised.

The composition obtained was in the form of a powder with good flow characteristics and uniform granulometry.

### Example N° 4 (comparison)

| Components | Quantity (g) |
|---|---|
| Cyclosporin | 2.89 |
| Tween 808® | 3.44 |
| Akoline® | 13.72 |

A solution of cyclosporin (2.89g) in Akoline^{®} (13.72g) has been firstly prepared with magnetic stirring, at a speed of 100 rpm for 4 hours. To the solution have then been added 3.44g of Tween 80^{®} and the agitation has been maintained for an hour.

The solution obtained has been finally incorporated into the cross-linked polymer crospovidone Kollidon CL^{®} in a weight ratio of 1.0:1.0 in a twin screw extruder APV (U.K.) and the product has then been spheronised.

The powder obtained had good flow characteristics, and uniform granulometry.

### Example N° 5

| Components of the o/w/o microemulsion | Quantity (g) |
|---|---|
| Cyclosporin | 0.569 |
| Labrafil®/Lecithin (5:1) (internal phase) | 0.118 |
| 0.1 % HCl aqueous solution | 0.326 |
| Tween 80® | 0.120 |
| Labrafil®/Lecithin (5:1) (external phase) | 8.01 |

An o/w microemulsion has been firstly prepared through mixing by magnetic stirring at a temperature of 25°C, a mixture of Labrafil®/lecithin (5:1) (0.118g) containing dissolved cyclosporin (0.083g), a 0,1% HCl aqueous solution (0.326g) and the surfacting agent Tween 80^{®} (0.120g) leaving with agitation for 2 hours at 200 rpm.

The o/w microemulsion has then been added with magnetic stirring at a speed of 500 rpm for 2 hours, to the solution of cyclosporin (0.486g) in Labrafil^{®}/lecithin (8.01 g) thus obtaining an o/w/o double microemulsion containing cyclosporin. The microemulsion thus obtained has been incorporated into crospovidone Kollidon Cl M® by granulation in a high efficiency granulator from Società Battagion (Bergamo-Italy). The weight ratio between the o/w/o microemulsion and the crospovidone was of 0.69:1:

The composition obtained was in the form of a powder having good flow characteristics and uniform granulometry.

### Example N° 6

| Components of the o/w/o microemulsion | Quantity |
|---|---|
| Hydrocortisone acetate | 5.0 |
| Akoline^{®} (internal phase) | 1.90 |
| Demineralised water | 6.00 |
| Tween 80^{®} | 2.40 |
| Akoline^{®} (external phase) | 48.0 |

An o/w microemulsion has been firstly prepared through mixing by magnetic stirring at a temperature of 25°C Akoline^{®} (1.80g) containing dissolved hydrorcortisone acetate (1.40g), demineralised water (6.00g) and Tween 80^{®} (2.40g), maintaining the agitation at a speed of 500 rpm for two hours.

The o/w microemulsion thus obtained has then been added with magnetic stirring at a speed of 500 rpm to a solution of hydrocortisone acetate (3.60g) in Akoline (48.0g) and the agitation has been maintained for 3 hours, obtaining an o/w/o double microemulsion containing hydrocortisone acetate.

The microemulsion thus obtained has been incorporated into microporous silica Syloid^{®} by granulation in a high efficiency granulator from Società Battagion (Bergamo-Italy).

The weight ratio between the o/w/o microemulsion and the microporous silica is of 2.0;1.0.

The composition obtained was in the form of a powder having good flow characteristics and uniform granulometry.

### Example N° 7 (comparison)

| Components of the o/w microemulsion | Quantity (g) |
|---|---|
| Hydrocortisone acetate | 0.56 |
| Akoline (internal phase) | 3.18 |
| Demineralised water | 4.4 |
| Tween 80^{®} | 1.90 |

An oil/water microemulsion has been firstly prepared through mixing by magnetic stirring at temperature of 25°C Akoline^{®} (3.18g) containing dissolved hydrocortisone acetate (0.56g), demineralised water (4.4g) and Tween 80^{®} (1.9g), maintaining the magnetic stirring at a speed of 500 rpm for 2 hours.

The microemulsion thus obtained has been incorporated into microporous silica Syloid^{®} by granulation in a high efficiency granulator from Società Battagion (Bergamo-Italy).

The weight ratio between the o/w microemulsion and the microporous silica was of 1.0:1.0.

Example N° 8

| Components of the o/w/o microemulsion | Quantity (g) |
|---|---|
| Ubidecarenone | 0.30 |
| Labrafil^{®}/Lecithin (5:1) (internal phase) | 0.130 |
| 0.1 % HCl aqueous solution | 0.335 |
| Tween 80^{®} | 0.120 |
| Labrafil^{®}/Lecithin (5:1) (external phase) | 8.30 |

An o/w microemulsion has been firstly prepared through mixing by magnetic stirring at a temperature of 25°C the Labrafil^{®}/lecithin mixture (5:1) (0.130g) containing dissolved Ubidecarenone (0.065g) with the aqueous solution containing 0.1 % HCl (0.335g) and Tween 80^{®} (0.120g), maintaining the magnetic stirring at a speed of 350 rpm for 2 hours.

The o/w microemulsion thus obtained has then been added with magnetic stirring at a speed of 500 rpm to a solution of Ubidecarenone (0.235g) in Labrafil/Lecithin (8.30g) and the agitation has been maintained for 4 hours, obtaining an o/w/o double microemulsion containing Ubidecarenone.

The microemulsion thus obtained has been incorporated into the cross-linked polymer crospovidone Kollidon CL^{®} in the weight ratio 2.2:1.0 in a twin screw extruder APV (U.K.) And the product has then been spheronised.

A product has been obtained in microgranular form.

### Example N° 9 (comparison)

| Components of the o/w microemulsion | Quantity (g) |
|---|---|
| Ubidecarenone | 2.09 |
| Labrafil^{®}/Lecithin (5:1) (internal phase) | 2.07 |
| 0.1% HCl aqueous solution | 5.87 |
| Tween 80^{®} | 2.00 |

An o/w microemulsion has been prepared through mixing by magnetic stirring at a temperature of 25°C the Labrafil^{®}/Lecithin mixture (5:1) (2.07g) containing dissolved Ubidecarenone (2.09g), the aqueous solution containing 0.1 % HCl (5.87g) and Tween 80^{®} (2.00g), maintaining the magnetic stirring at a speed of 400 rpm for 3 hours.

The microemulsion thus obtained has been incorporated into the cross-linked polymer crospovidone Kollidon CL^{®} in a weight ratio of 1.0:1.0 in a twin screw extruder APV (U.K.) and the product has then been spheronised.

A product has been obtained in microgranular form.

## Claims

1. A pharmaceutical composition in powder or microgranular form, comprising a poorly soluble drug, comprising an oil/water/oil double microemulsion incorporated into a solid support constituted by a microporous inorganic substance or by an adsorbent colloidal inorganic substance or by a cross-linked swellable in water polymer, wherein said drug is dissolved or dispersed in one or more of the phases of said microemulsion.

2. The composition according to claim 1, **characterised by** the fact that said microemulsion has the following composition by weight.
| | |
|---|---|
| oil (internal phase) | from 1,5% to 3.0% |
| water or aqueous solution | from 6.1 % to 10.0% |
| Surfactant | from 2.4% to 2.0% |
| Cosurfactant | from 0.0% a 2.0% |
| oil (external phase) | from 80.0% to 90.0% |

3. The composition according to claim 1, **characterised by** the fact that said drug is chosen from the group comprising megestrol acetate, hydrocortisone acetate, ubidecarenone, lovastatin, cyclosporin, pyroxican, nifedipine, isoflavone, temazepam, carbamazepine, glibenclamide, progesterone and ibuprofen.

4. The composition according to claim 1, **characterised by** the fact that said oil is selected from the group comprising olive oil, soya oil, corn oil, coconut oil, isopropyl myristate, isopropyl palmitate, ethyl laurate, fatty acids, mixtures of mono-, di- and tri-glycerides and derivatives thereof esterified with polyethyleneglycols.

5. The composition according to claim 1, **characterised by** the fact that said surfactants are selected from the group comprising Tween^{®}, Brij^{®}, Span^{®}, Myrj^{®} and Polaxamer^{®}.

6. The composition according to claim 1, **characterised by** the fact that said cosurfactants are selected from the group comprising ethanol, isopropanol, phosphatidylcholine, phosphatidylethanolamine and phosphatidylglycerol.

7. The composition according to claim 1, **characterised by** the fact that said microporous inorganic substance is selected from the group comprising silica, silicates, zeolytes, allumina and activated carbon.

8. The composition according to claim 1, **characterised by** the fact that said adsorbent, colloidal inorganic substance is selected from the group comprising colloidal silica, magnesium trisilicate, argil, magnesium hydroxide and talc.

9. The compositions according to claim 1, **characterised by** the fact that said cross-linked swellable in water polymers are selected from the group comprising polyvinylpyrrolidone, cross-linked sodium carboxymethylstarch, cross-linked sodium carboxymethylcellulose, cross-linked polystyrene and cross-linked polymethylmetacrylate.

10. The composition according to claim 1, **characterised by** the fact that the weight ratio between said microemulsion and said solid support is comprised of between 1:100 and 25:1.

11. The composition according to claim 1, **characterised by** the fact that the weight ratio between said microemulsion and said solid support is comprised of between 1:2 and 5:1.

12. The composition according to claim 1, **characterised by** the fact that the drug content is comprised of between 0.001 % and 75%.

13. The composition according to claim 1, **characterised by** the fact that the drug content is comprised of between 0.01 % and 30%.

14. The composition according to claim 1, formulated with pharmaceutically acceptable excipients or diluents, for use in capsules, pills, sachets and suspensions.

15. A process for the preparation of a pharmaceutical composition such as defined in claim 1, **characterised by** the fact of comprising the following stages:
a) dissolution of the drug in an oil or in a mixture of oils;
b) addition of the oil solution of stage a) to water or to an aqueous solution;
c) addition of a surfactant and optionally of a cosurfactants to the mixture of stage b) and agitation with the formation of an o/w microemulsion;
d) addition of the o/w microemulsion of stage c) to an oil or to a mixture of oils optionally containing drug and/or surfactant and/or cosurfactant and agitation with formation of the o/w/o microemulsion;
e) incorporation of the o/w/o microemulsion of stage d) into a support in the form of a powder.

16. The process according to claim 15, **characterised by** the fact that said oil or mixture of oils contains also a drug in the form of a suspension.

17. The process according to claim 15, **characterised by** the fact that said support in the form of a powder is preliminarily loaded with a drug.

18. The process according to claim 15, **characterised by** the fact that said incorporation is carried out by slowly adding said microemulsion to said support in powder form, maintaining said support under constant mixing/agitation in an equipment selected from high efficiency of mixing granulators, extruders and fluid bed granulators.

## Patentansprüche

1. Pharmazeutische Zusammensetzung in Pulverform oder mikrogranulärer Form, umfassend ein schwach lösliches Medikament, umfassend eine doppelte Öl/Wasser/Öl-Mikroemulsion, die in einen festen Träger eingeschlossen ist, welcher aus einer mikroporösen anorganischen Substanz oder einer adsorbierenden kolloidalen anorganischen Substanz oder aus einem vernetzten in Wasser aufquellbaren Polymer besteht, wobei das Medikament in einer oder mehreren der Phasen dieser Mikroemulsion gelöst oder dispergiert ist.

2. Zusammensetzung gemäß Anspruch 1, **dadurch gekennzeichnet, daß** diese Mikroemulsion die folgende Zusammensetzung nach Gewicht hat:
| | |
|---|---|
| Öl (interne Phase) | von 1,5 bis 3,0 % |
| Wasser oder wäßrige Lösung | von 6,1 bis 10,0 % |
| Tensid | von 2,4 bis 2,0 % |
| Cotensid | von 0,0 bis 2,0 % |
| Öl (externe Phase) | von 80,0 bis 90,0 % |

3. Zusammensetzung gemäß Anspruch 1, **dadurch gekennzeichnet, daß** das Medikament ausgewählt ist aus der Gruppe, die aus Megestrolacetat, Hydrocortisonacetat, Ubidecarenon, Lovastatin, Cyclosporin, Pyroxican, Nifedipin, Isoflavon, Temazepam, Carbamazepin, Glibenclamid, Progesteron und Ibuprofen besteht.

4. Zusammensetzung gemäß Anspruch 1, **dadurch gekennzeichnet, daß** das Öl aus der Gruppe ausgewählt aus ist, die Olivenöl, Sojabohnenöl, Maisöl, Kokosnußöl, Isopropylmyristat, Isopropylpalmitat, Ethyllaurat, Fettsäuren, Mischungen aus Mono-, Di- und Triglyceriden und Derivaten davon, die mit Polyethylenglycolen verestert sind, umfaßt.

5. Zusammensetzung gemäß Anspruch 1, **dadurch gekennzeichnet, daß** die Tenside aus der Gruppe ausgewählt sind, die Tween^{®}, Brij^{®}, Span^{®}, Myrj^{®} und Polaxamer^{®} umfaßt.

6. Zusammensetzung gemäß Anspruch 1, **dadurch gekennzeichnet, daß** die Cotenside aus der Gruppe ausgewählt werden, die Ethanol, Isopropanol, Phosphatidylcholin, Phosphatidylethanolamin und Phosphatidylglycerol umfaßt.

7. Zusammensetzung gemäß Anspruch 1, **dadurch gekennzeichnet, daß** die mikroporöse anorganische Substanz aus der Gruppe ausgewählt ist, die Kieselsäure, Silicate, Zeolyte, Tonerde und Aktivkohle umfaßt.

8. Zusammensetzung gemäß Anspruch 1, **dadurch gekennzeichnet, daß** die adsorbierende kolloidale anorganische Substanz aus der Gruppe ausgewählt ist, die kolloidale Kieselsäure, Magnesiumtrisilicat, Argil, Magnesiumhydroxid und Talk umfaßt.

9. Zusammensetzungen gemäß Anspruch 1, **dadurch gekennzeichnet, daß** die vernetzten in Wasser aufquellbaren Polymere aus der Gruppe ausgewählt werden, die Polyvinylpyrrolidon, vernetze Natriumcarboxymethylstärke, vernetzte Natriumcarboxymethylcellulose, vernetztes Polystyrol und vernetztes Polymethylmethacrylat umfaßt.

10. Zusammensetzung gemäß Anspruch 1, **dadurch gekennzeichnet, daß** das Gewichtsverhältnis zwischen der Mikroemulsion und dem festen Träger von zwischen 1:100 und 25:1 umfaßt ist.

11. Zusammensetzung gemäß Anspruch 1, **dadurch gekennzeichnet, daß** das Gewichtsverhältnis zwischen der Mikroemulsion und dem festen Träger von zwischen 1:2 und 5:1 umfaßt ist.

12. Zusammensetzung gemäß Anspruch 1, **dadurch gekennzeichnet, daß** der Medikamentenanteil von zwischen 0,001 % und 75 % umfaßt ist.

13. Zusammensetzung gemäß Anspruch 1, **dadurch gekennzeichnet, daß** der Medikamentenanteil von zwischen 0,01 und 30 % umfaßt ist.

14. Zusammensetzung gemäß Anspruch 1, die mit pharmazeutisch annehmbaren Exzipienten oder Verdünnungsmitteln zur Verwendung in Kapseln, Pillen, Portionspackungen und Suspensionen formuliert ist.

15. Verfahren zur Herstellung einer pharmazeutischen Zusammensetzung wie in Anspruch 1 beschrieben, **dadurch gekennzeichnet, daß** es die folgenden Schritte umfaßt:
a) Lösen des Medikaments in einem Öl oder in einer Mischung aus Ölen;
b) Zugabe der Öllösung aus Schritt a) zu Wasser oder zu einer wäßrigen Lösung;
c) Zugabe eines Tensids und gegebenenfalls von Cotensiden zu der Mischung aus Schritt b) und Rühren unter Ausformung einer o/w-Mikroemulsion;
d) Zugabe der o/w-Mikroemulsion des Schritts c) zu einem Öl oder zu einer Mischung aus Ölen, die gegebenenfalls ein Medikament und/oder Tensid und/oder Cotensid enthalten, und Rühren unter Ausbildung der o/w/o-Mikroemulsion;
e) Einschließen der o/w/o-Mikroemulsion des Schritts d) in einem Träger in Form eines Pulvers.

16. Verfahren gemäß Anspruch 15, **dadurch gekennzeichnet, daß** das Öl oder die Mischung aus Ölen auch ein Medikament in Form einer Suspension enthält.

17. Verfahren gemäß Anspruch 15, **dadurch gekennzeichnet, daß** der Träger in Form eines Pulvers vorher mit einem Medikament beladen wurde.

18. Verfahren gemäß Anspruch 15, **dadurch gekennzeichnet, daß** das Einschließen durch langsames Zugeben der Mikroemulsion zu dem Träger in Pulverform durchgeführt wird, wobei der Träger unter kontinuierlicher Durchmischung/Rühren in einer Vorrichtung belassen wird, die aus Granulatoren, Extrudern und Flüssigbettgranulatoren mit hoher Effizienz ausgewählt sind.

## Revendications

1. Composition pharmaceutique sous forme pulvérulente ou microgranulaire, comprenant un médicament faiblement soluble comprenant une double microémulsion huile/eau/huile incorporée dans un support solide constitué d'une substance inorganique microporeuse ou d'une substance inorganique colloïdale adsorbante ou d'un polymère réticulé gonflable dans l'eau, dans laquelle ledit médicament est dissous ou dispersé dans une ou plusieurs des phases de ladite microémulsion.

2. Composition selon la revendication 1, **caractérisée en ce que** ladite microémulsion a la composition suivante en poids.
| | |
|---|---|
| Huile (phase interne) | de 1,5 % à 3,0 % |
| Eau ou solution aqueuse | de 6,1 % à 10,0 % |
| Agent tensioactif | de 2,4 % à 2,0 % |
| Coagent tensioactif | de 0,0 % à 2,0 % |
| Huile (phase externe) | de 80,0 % à 90,0 % |

3. Composition selon la revendication 1, **caractérisée en ce que** ledit médicament est choisi dans le groupe comprenant l'acétate de mégestrol, l'acétate d'hydrocortisone, l'ubidécarénone, la lovastatine, la cyclosporine, le pyroxican, la nifédipine, l'isoflavone, le témazépam, la carbamazépine, le glibenclamide, la progestérone et l'ibuprofène.

4. Composition selon la revendication 1, **caractérisée en ce que** ladite huile est choisie dans le groupe comprenant l'huile d'olive, l'huile de soja, l'huile de maïs, l'huile de noix de coco, le myristate d'isopropyle, le palmitate d'isopropyle, le laurate d'éthyle, les acides gras, les mélanges de mono-, di- et triglycérides et des dérivés de ceux-ci estérifiés avec des poly(éthylène glycols).

5. Composition selon la revendication 1, **caractérisée en ce que** lesdits agents tensioactifs sont choisis dans le groupe comprenant Tween^{®}, Brij^{®}, Span^{®}, Myrj^{®} et Polaxamer^{®}.

6. Composition selon la revendication 1, **caractérisée en ce que** lesdits coagents tensioactifs sont choisis dans le groupe comprenant l'éthanol, l'isopropanol, la phosphatidylcholine, la phosphatidyléthanolamine et le phosphatidylglycérol.

7. Composition selon la revendication 1, **caractérisée en ce que** ladite substance inorganique microporeuse est choisie dans le groupe comprenant la silice, les silicates, les zéolites, l'alumine et le charbon actif.

8. Composition selon la revendication 1, **caractérisée en ce que** ladite substance inorganique colloïdale adsorbante est choisie dans le groupe comprenant la silice colloïdale, le trisilicate de magnésium, l'argile, l'hydroxyde de magnésium et le talc.

9. Composition selon la revendication 1, **caractérisée en ce que** lesdits polymères réticulés gonflables dans l'eau sont choisis dans le groupe comprenant la poly(vinylpyrrolidone), le carboxyméthylamidon sodique réticulé, la carboxyméthylcellulose sodique réticulée, le poly(styrène) réticulé et le poly(méthacrylate de méthyle) réticulé.

10. Composition selon la revendication 1, **caractérisée en ce que** le rapport en poids entre ladite microémulsion et ledit support solide est compris entre 1: 100 et 25: 1.

11. Composition selon la revendication 1, **caractérisée en ce que** le rapport en poids entre ladite microémulsion et ledit support solide est compris entre 1: 2 et 5: 1.

12. Composition selon la revendication 1, **caractérisée en ce que** la teneur en médicaments est comprise entre 0,001 % et 75 %.

13. Composition selon la revendication 1, **caractérisée en ce que** la teneur en médicaments est comprise entre 0,01 % et 30 %.

14. Composition selon la revendication 1, formulée avec des excipients ou des diluants pharmaceutiquement acceptables, pour une utilisation dans des capsules, des pilules, des sachets et des suspensions.

15. Procédé de préparation d'une composition pharmaceutique selon la revendication 1, **caractérisé en ce qu'**il comprend les étapes suivantes:
a) dissolution du médicament dans une huile ou dans un mélange d'huiles;
b) addition de la solution d'huile de l'étape a) à de l'eau ou à une solution aqueuse;
c) addition d'un agent tensioactif et facultativement des coagents tensioactifs au mélange de l'étape b) et agitation avec la formation d'une microémulsion huile/eau;
d) addition de la microémulsion huile/eau de l'étape c) à une huile ou à un mélange d'huiles contenant facultativement un médicament et/ou un agent tensioactif et/ou un coagent tensioactif et agitation avec formation de la microémulsion huile/eau/huile;
e) incorporation de la microémulsion huile/eau/huile de l'étape d) dans un support sous la forme d'une poudre.

16. Procédé selon la revendication 15, **caractérisé en ce que** ladite huile ou ledit mélange d'huiles contient également un médicament sous la forme d'une suspension.

17. Procédé selon la revendication 15, **caractérisé en ce que** ledit support sous la forme d'une poudre est au préalable chargé avec un médicament.

18. Procédé selon la revendication 15, **caractérisé en ce que** ladite incorporation est réalisée en ajoutant lentement ladite microémulsion audit support sous forme pulvérulente, en maintenant ledit support sous un mélange/agitation constant dans un équipement choisi à partir d'un rendement élevé de granulateurs de mélange, d'extrudeuses et de granulateurs à lit fluidisé.
